# FASCICULE DE BREVET EUROPEEN

(11) **EP 1 843 855 B1**
(45) Date de publication et mention de la délivrance du brevet: **21.12.2011**
(21) Numéro de dépôt: 06700532.2
(22) Date de dépôt: 23.01.2006
(51) Int. Cl.: B04B 5/04, B04B 11/08, B04B 9/12, B04B 7/00, B04B 5/10

(54) **SEPARATEUR CENTRIFUGE POUR LIQUIDE PHYSIOLOGIQUE EN PARTICULIER POUR LE SANG**
ZENTRIFUGALABSCHEIDER FÜR EINE PHYSIOLOGISCHE FLÜSSIGKEIT, INSBESONDERE BLUT
CENTRIFUGAL SEPARATOR FOR A PHYSIOLOGICAL LIQUID, PARTICULARLY BLOOD

(30) Priorité: 25.01.2005 EP 05405037
(43) Date de publication de la demande: 17.10.2007
(73) Titulaire: Rochat, Jean-Denis, 1272 Genolier (CH)
(72) Inventeur: Rochat, Jean-Denis, 1272 Genolier (CH)
(74) Mandataire: Moinas, Michel
(86) Numéro de dépôt international: PCT/CH2006/000048
(87) Numéro de publication internationale: WO 2006/079237

(56) Documents cités:
- EP-A- 0 297 216
- EP-A- 1 057 534
- DE-C1- 19 822 191
- US-A- 2 043 313
- US-A- 5 851 169
- PATENT ABSTRACTS OF JAPAN vol. 1997, no. 11, 28 novembre 1997 (1997-11-28) -& JP 09 192215 A (NAKANE TAKAHARU), 29 juillet 1997 (1997-07-29) cité dans la demande
- PATENT ABSTRACTS OF JAPAN vol. 006, no. 054 (C-097), 9 avril 1982 (1982-04-09) -& JP 56 166957 A (ASAHI CHEM IND CO LTD), 22 décembre 1981 (1981-12-22)

## Description

La présente invention se rapporte à un séparateur centrifuge pour un fluide physiologique, en particulier pour le sang, comprenant un conduit fixe pour alimenter en continu une enceinte jetable rigide de centrifugation en liquide à séparer, deux conduits fixe de sortie dont les ouvertures d'admission respectives se situent dans deux zones de concentration des constituants séparés pour les retirer en continu, ces conduits fixes étant solidaires d'une partie fixe de ladite enceinte, des moyens de guidage en rotation de cette enceinte, des moyens d'entraînement de support et de guidage en prise avec un élément d'accouplement axial solidaire du fond de ladite enceinte et des moyens de positionnement de la partie fixe de ladite enceinte par rapport aux moyens d'entraînement de support et de guidage.

On connaît déjà des séparateurs de ce type travaillant en semi-continu, dans lesquels l'enceinte de centrifugation est constituée par un organe jetable. Par conséquent, parmi les éléments entrant dans l'appréciation des avantages d'un séparateur, outre ses performances de séparation, le débit moyen de sang traité et le coût du consommable constituent deux facteurs importants. En ce qui concerne les performances, le taux d'hémolyse et d'activation est un autre facteur important, qui dépend notamment des forces appliquées aux constituants du sang, notamment aux globules rouges et aux plaquettes et de la durée pendant laquelle ces constituants sont soumis aux forces destinées à les séparer des autre composants du sang. Plus cette durée est réduite plus le taux d'hémolyse diminue. C'est ainsi qu'une force relativement élevée peut être appliquée pendant une courte durée, alors qu'une force sensiblement plus faible provoque un taux d'hémolyse plus élevé lorsqu'elle est appliquée sur une période sensiblement plus longue. Par conséquent, les enceintes de centrifugation connues ont des volumes relativement grands, augmentant le temps de séjours des constituants du sang dans l'enceinte de centrifugation et donc le taux d'hémolyse.

Le coût du consommable est notamment en relation avec son volume et celui-ci détermine directement l'encombrement de l'installation de séparation et donc aussi son coût.

Les forces exercées dépendent de la vitesse de rotation et du rayon de l'enceinte de séparation.

Dans l'immense majorité des cas, les séparateurs existants utilisent des bols ou cuvettes de centrifugation de diamètres relativement grands, qui sont très souvent plus grands que la dimension axiale du bol ou de la cuvette.

Plusieurs séparateurs centrifuge de plasmaphérèse travaillant en semi-continu utilisent le principe divulgué par le US 4'300'717, dans lequel le bol de centrifugation est formé par une cloche, l'écoulement du sang en cours de séparation s'effectuant entre un noyau central et la paroi latérale de la cloche, les globules rouges étant retenue entre la cloche et le noyau alors que le plasma est évacué par une sortie située au sommet de la cloche.

On a utilisé ce même principe, mais en supprimant le noyau central dans le EP 0'608'519.

Dans les séparateurs susmentionnés, les bols de centrifugation ne sont pas guidés de manière précise, en sorte que le joint tournant doit être conçu pour permettre non seulement d'assurer l'étanchéité mais aussi de compenser le décentrage de l'enceinte de centrifugation qui peut être de plusieurs dixièmes de mm autour de son axe de rotation. Dans le contexte d'un système travaillant en semi-continu, dans lequel les globules rouges sont stockées dans le bol de centrifugation, ce décentrage est tolérable.

Parmi les séparateurs travaillant en continu, la plupart utilisent un tube souple pour l'alimentation du sang à séparer et l'évacuation de ses constituants séparés. On peut citer notamment le US 5'750'025, le US 5'350'514, le US 5'628'915 ou le US 5'851'169. Dans deux cas, l'enceinte de séparation jetable est constituée par une poche souple destinée à être introduite sur un support rotatif rigide. La mise en place sur ce support d'une telle poche, munie d'une pluralité de conduits souples, constitue une opération longue et délicate.

Le système utilisé pour annuler l'effet de la rotation de l'enceinte de centrifugation sur la fixation du conduit souple à cette enceinte, dans les séparateurs centrifuge de ce type, est divulgué dans le US 3'586'413. Il permet, en formant une boucle ouverte dont une extrémité est solidaire en rotation de l'axe de la cuvette de centrifugation tournant à la vitesse 2ω, tandis que son autre extrémité, coaxiale à la première, est fixe alors que la boucle ouverte est entraînée à la vitesse ω, d'engendrer une rotation du tube souple tournant autour de son propre axe à la vitesse -ω et d'annuler ainsi toute torsion du tube souple.

Ce principe, qui permet de supprimer tout joint entre le tube souple et l'organe tournant a été largement repris dans un grand nombre de dispositifs de centrifugation travaillant à écoulement continu. En effet, contrairement aux centrifugeuses à tube d'alimentation et d'évacuation fixe, les composants séparés ne subissent pas une décélération brutale de leur vitesse tangentielle, en sorte que les risques d'hémolyse sont réduits.

Toutefois, compte tenu de la vitesse de l'organe tournant dans une centrifugeuse, le tube souple tournant sur lui-même à la vitesse -ω est soumis, à une contrainte de traction engendrée par la force centrifuge, à une contrainte de flexion due à la rotation sur lui-même de la portion du tube formant la boucle ouverte à la vitesse -ω, ainsi qu'à un échauffement engendré par le travail des forces visqueuses dans la matière due à la flexion susmentionnée. Or, dans le cas de la centrifugation de sang, sa température ne doit être > 40°C.

Par conséquent la vitesse de rotation de la cuvette de centrifugation est limitée, en sorte que le diamètre de cette cuvette ne peut pas être trop petit sous peine de nuire à la qualité de la séparation. En outre, le mécanisme d'entraînement de la cuvette et du tube souple est relativement complexe et coûteux.

On a encore proposé dans le JP 09 192215 et dans le EP 0 297 216, des séparateurs centrifuges comprenant une cuvette de centrifugation conique rigide, dont l'alimentation et l'évacuation des composants séparés est réalisée par des conduits fixes engagés dans une ouverture axiale supérieure de la cuvette. Cette cuvette est entraînée par friction et guidée par un disque d'entraînement muni d'une ouverture axiale dans laquelle un élément cylindrique faisant saillie du fond de la cuvette est engagé. Un joint glissant en forme de soufflet est disposé entre les conduits fixes et le sommet de la cuvette conique. Le EP 0 297 216 ne comporte aucun guidage de la partie supérieure de la cuvette, alors que dans le JP 09 192215, c'est le bord d'une calotte de protection du joint glissant qui s'appuie sur une surface conique du sommet de la cuvette.

On a proposé dans le DE 198 22 191 de guider une cuvette de centrifugation par trois galets solidaires d'une bague coulissante et dont les axes respectifs sont parallèles à une surface de guidage conique de l'axe de rotation de la cuvette. La bague coulissante est guidée par une surface cylindrique parallèle à l'axe de rotation de la cuvette. Le déplacement axial des galets de guidage ne constitue généralement pas une solution appropriée aux enceintes de centrifugation associées à des conduits fixes traversant une ouverture axiale de l'extrémité supérieure de ces enceintes. En outre, rien n'est prévu pour maintenir les galets solidaires de la bague coulissante appliqués contre la surface de guidage conique, en sorte que la rotation de l'enceinte peut provoquer un déplacement axial de la bague coulissante et ne garantit ainsi pas un guidage correct de l'enceinte de centrifugation.

Il existe également une enceinte de centrifugation du document US 5851169 qui comporte un tube d'entrée d'air fixe, une tête centrifuge tournant autour du tube et expulsant l'air par un tube tournant, la tête centrifuge comprenant un collier qui porte un roulement à billes. Le roulement à billes est situé entre l'enceinte de centrifugation et le tube central et non pas entre le support et l'enceinte de centrifugation. Il ne permet donc pas un guidage rotatif de l'enceinte mais uniquement son pivotement.

On peut donc constater que les solutions existantes ne permettent pas de répondre de manière satisfaisante à la demande d'un séparateur simple, peu volumineux, facile à utiliser, travaillant avec des enceintes de centrifugations jetables bon marché, dans lesquelles le sang à traiter séjourne un minimum de temps et apte à travailler à un bon débit.

C'est la raison pour laquelle il est apparu nécessaire de reconsidérer le concept du séparateur pour pouvoir répondre de manière plus satisfaisante aux exigences susmentionnées.

Le but de la présente invention est de remédier, au moins en partie, aux inconvénients susmentionnés.

A cet effet, la présente invention a pour objet un séparateur centrifuge selon la revendication 1.

L'avantage de ce séparateur centrifuge est de travailler en écoulement continu avec des conduits fixes, ce qui est admissible pour autant que la force tangentielle ne dépasse pas une certaine limite pour ne pas soumettre les globules rouges et les plaquettes à une décélération trop brutale lorsqu'elle passent de l'enceinte de centrifugation dans le conduit d'évacuation fixe.

Comme on l'a mentionné précédemment, les forces auxquelles le globules rouges ou les plaquettes sont soumises ne constituent pas le seul facteur d'hémolyse ou d'activation. La durée pendant laquelle ces constituants du sang sont soumis à des forces mêmes modérées est un autre facteur d'hémolyse ou d'activation.

C'est la raison pour laquelle, de préférence, le séparateur objet de cette invention permet d'assurer un guidage précis de l'enceinte de centrifugation de type jetable. Une enceinte de ce type doit être en matière plastique rigide. Par «rigide», on entend une enceinte apte à être entraînée et guidée directement par les moyens d'entraînement et de guidage du séparateur et susceptible de résister aux forces centrifuges auxquelles elle est soumise. Pour être à même d'obtenir un guidage précis d'une telle enceinte, il faut être aussi peu dépendant que possible des erreurs dues au retrait inhérent de la matière plastique et aux tolérances du moule. On sait en effet, que toute matière plastique subit un retrait lors de son refroidissement, en sorte que plus on travaille sur un grand diamètre plus l'erreur augmente, le retrait étant proportionnel à la dimension de la pièce.

Un autre facteur est à prendre en considération en utilisant des conduits fixes pour l'alimentation et l'évacuation en continu de l'enceinte de centrifugation. On doit utiliser une enceinte avec un joint frottant entre la partie fixe d'alimentation et d'évacuation et la partie tournante de centrifugation. Il est alors avantageux de travailler sur un diamètre aussi réduit que possible pour diminuer l'échauffement. Un guidage précis permet aussi de réduire au minimum la précontrainte à laquelle le joint doit être soumis, dans la mesure où sa seule fonction est alors d'assurer l'étanchéité et non plus les erreurs dues à l'imprécision de guidage, comme c'est le cas des enceintes connues de centrifugation à conduits fixes d'alimentation et d'évacuation.

Le guidage précis de l'enceinte de centrifugation dans le séparateur centrifuge, dans le cas d'une séparation à écoulement continu, permet de travailler sur une épaisseur de liquide très faible sur la paroi de l'enceinte de centrifugation et donc de diminuer le temps de séjour du liquide, réduisant ainsi la durée pendant laquelle les constituants du sang sont soumis aux forces de séparation et diminuant donc, les risques d'hémolyse et d'activation des plaquettes.

Ce guidage sera d'autant plus précis que la distance axiale entre les deux guidages axiaux de l'enceinte de centrifugation est de préférence grande. Dès lors, on peut aussi de préférence, envisager avec profit de réduire le diamètre de l'enceinte de centrifugation et de la faire tourner plus vite. Ceci est rendu possible grâce au guidage axial de l'enceinte d'une part par son fond qui prend simplement appui sur l'arbre du moteur d'entraînement et d'autre part, par sa surface de guidage supérieure de diamètre sensiblement plus petit que celui de l'enceinte de centrifugation, venant en prise avec des galets de guidage. Ce guidage précis et l'utilisation de petits diamètre permettent de réduire l'échauffement au niveau du joint tournant. Le diamètre de l'enceinte de centrifugation peut avantageusement être faible puisqu'elle peut tourner plus vite. Par conséquent son volume, donc son prix peuvent sensiblement diminuer de même que les dimensions et l'encombrement du séparateur centrifuge.

Il ressort de ce qui précède que le nouveau concept basé sur une enceinte de centrifugation à conduits fixes et sur son guidage de précision, permet de concevoir un séparateur centrifuge à débit continu beaucoup moins volumineux que les séparateurs usuels de ce type à conduits rotatifs souples.

Le dessin annexé illustre, schématiquement et à titre d'exemple, une forme d'exécution du séparateur objet de la présente invention.
La figure 1 est une vue en élévation de face de cette forme d'exécution;
la figure 2 est une vue en perspective partielle de la figure 1;
la figure 3 est une vue de dessus de la figure 2;
la figure 4 est une vue partielle en coupe axiale, à plus grande échelle de la première forme d'exécution d'une enceinte de centrifugation;
la figure 5 est une vue semblable à la figure 4 d'une deuxième forme d'exécution d'une enceinte de centrifugation.

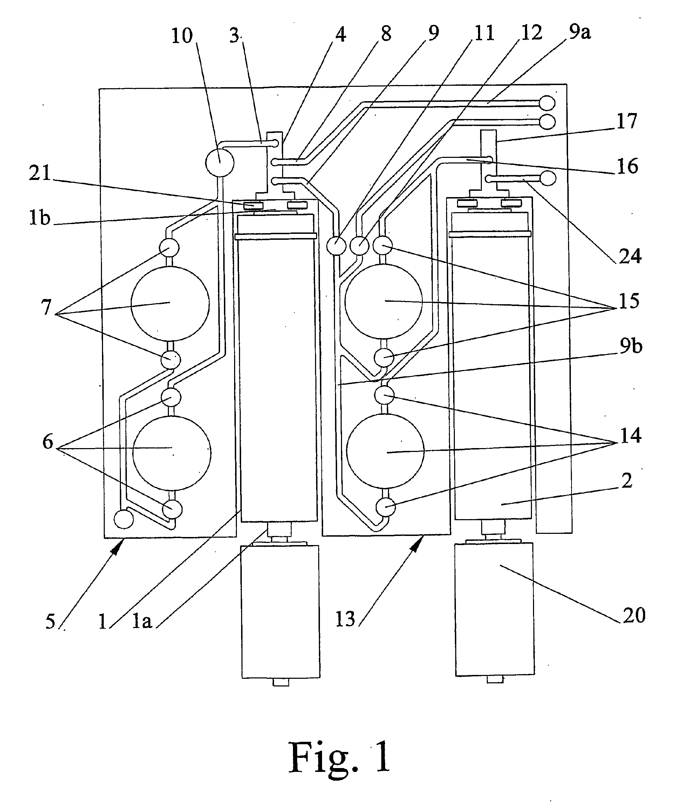

Le boîtier du séparateur centrifuge destiné à utiliser le dispositif selon la présente invention est illustré schématiquement par la figure 1 comporte deux enceintes allongées de centrifugation 1, 2 de forme tubulaire. La première enceinte tubulaire de centrifugation 1 comporte un conduit d'alimentation 3 qui est connecté à un élément axial fixe 4 d'entrée et de sortie de l'enceinte de centrifugation 1. Ce conduit d'alimentation 3 est relié à un dispositif de pompage 5 qui comporte deux pompes 6 et 7 déphasées de 180° l'une par rapport à l'autre pour assurer un débit continu d'un liquide physiologique, notamment de sang. Un détecteur d'air 10 est disposé le long du conduit d'alimentation 3.

Deux conduits de sortie 8, 9 sont connectés à l'élément axial fixe 4, pour permettre la sortie en continu de deux constituants de densités différentes du liquide physiologique. Dans le cas du sang, le conduit de sortie 8 est destiné à la sortie des globules rouges concentrées RBC et le conduit 9 à la sortie du plasma riche en plaquettes PRP. Ce conduit de sortie 9 comporte une valve 11 et se divise en deux branches 9a, 9b. La branche 9a sert à récupérer le concentré plaquettaire et est contrôlée par une valve 12. Les valves 11 et 12 fonctionnent en logique OU exclusif soit pour faire passer le PRP de l'enceinte 1 à l'enceinte 2, soit pour vider le concentré plaquettaire de l'enceinte 2 vers la sortie 9a. La branche 9b sert à conduire le PRP vers un dispositif de pompage 13 comportant deux pompes 14 et 15 déphasées de 180° et servant à assurer l'alimentation continue de la seconde enceinte tubulaire de centrifugation 2 par un conduit d'alimentation 16 connecté à un élément axial fixe 17 de la seconde enceinte tubulaire de centrifugation 2. Un conduit de sortie 24 pour le plasma pauvre en plaquettes PPP est aussi connecté à l'élément axial fixe 17.

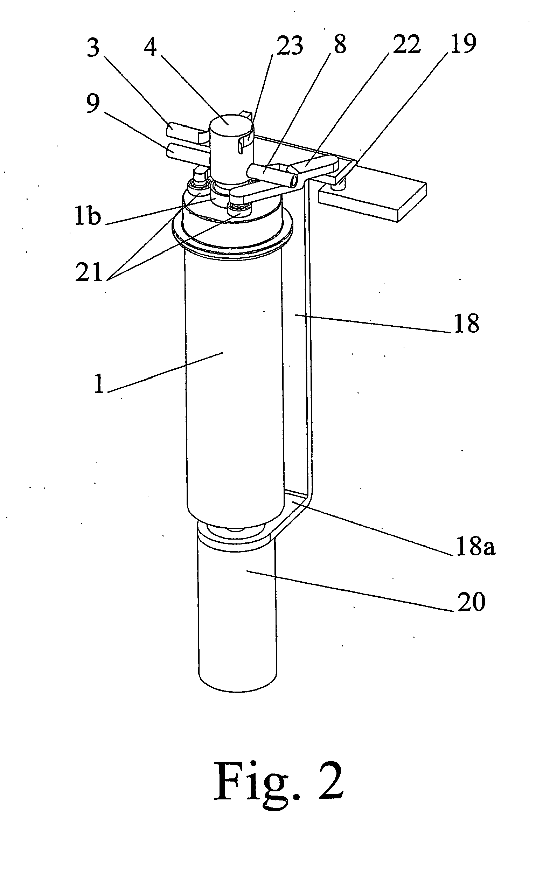

La figure 2 représente le mode d'entraînement et de guidage de l'enceinte tubulaire de centrifugation 1. L'ensemble des éléments d'entraînement et de guidage de l'enceinte tubulaire de centrifugation est situé sur un même support 18 relié au boîtier du séparateur centrifuge par une suspension anti-vibrations 19 de type silentbloc. Le support 18 présente une paroi verticale dont l'extrémité inférieure se termine par un bras horizontal de support 18a auquel est fixé un moteur d'entraînement 20. L'axe d'entraînement 20a de ce moteur 20 présente une forme polygonale, tel qu'un profil Torx^{®}, complémentaire d'un évidemment axial ménagé dans un petit élément tubulaire 1a qui fait saillie sous le fond de l'enceinte tubulaire de centrifugation 1. L'accouplement entre l'arbre d'entraînement du moteur 20 et l'élément tubulaire 1a doit être réalisé avec une très grande précision, pour assurer un guidage extrêmement précis de cette extrémité de l'enceinte tubulaire de centrifugation 1.

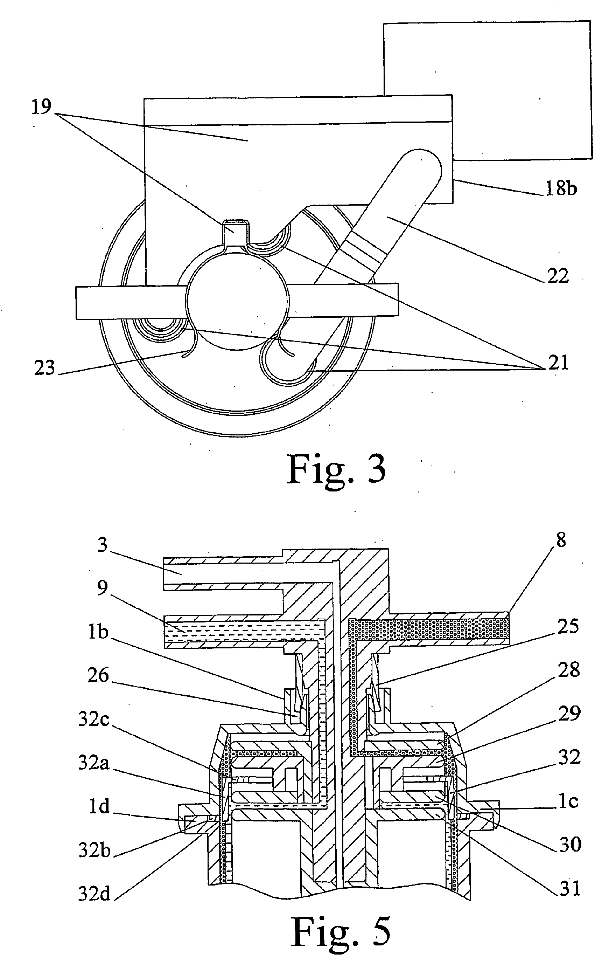

L'extrémité supérieure de l'enceinte tubulaire de centrifugation 1 comporte un élément cylindrique de guidage axial 1b de diamètre sensiblement plus faible que celui de l'enceinte tubulaire de centrifugation 1, qui fait saillie sur sa face supérieure. La face cylindrique de cet élément 1b est destinée à venir en prise avec trois galets de centrage 21 que l'on voit en particulier sur la figure 3. Un de ces galets 21 est solidaire d'un bras 22 dont une extrémité est montée pivotante sur une partie horizontale supérieure 18b du support 18. Ce bras 22 est soumis à la force d'un ressort (non représenté) ou tout autre moyen approprié, destiné à lui communiquer un couple tendant à le faire tourner dans le sens des aiguilles de la montre si l'on se réfère à la figure 3, en sorte qu'il appuie de manière élastique contre la surface cylindrique de l'élément cylindrique de guidage axial 1b, en sorte que l'enceinte tubulaire de centrifugation peut être mise en place et enlevée du support 18 en faisant pivoter le bras 22 dans le sens contraire à celui des aiguilles de la montre. Un dispositif de verrouillage de la position angulaire du bras 22 correspondant à celle dans laquelle son galet 21 appuie contre la surface cylindrique de l'élément cylindrique de guidage axial 1b est prévu, pour éviter d'avoir une précontrainte trop forte du ressort associé au bras 22.

La portée entre l'élément cylindrique de guidage axial 1b et l'extrémité supérieure de l'enceinte tubulaire 1 sert, en coopération avec les galets de centrage 21, de butée axiale, empêchant le désaccouplement entre l'axe d'entraînement du moteur 20 et l'évidemment axial de l'élément tubulaire 1a faisant saillie sous le fond le l'enceinte tubulaire 1.

Avantageusement, on pourrait aussi légèrement incliner les axes de rotation des galets de guidage 21 de quelques degrés angulaires, < 2° dans des plans respectifs tangents à un cercle coaxial à l'axe de rotation de l'enceinte tubulaire de centrifugation 1, passant par les axes de rotation respectifs des trois galets, dans un sens choisi, en fonction du sens de rotation des galets, dans lequel ceux-ci induisent sur l'enceinte tubulaire 1 une force dirigée vers le bas.

Un élément élastique de centrage et de fixation 23 de l'élément fixe axial 4 d'entrée et de sortie de l'enceinte tubulaire de centrifugation est solidaire de la partie horizontale supérieure 18b du support 18. Cet élément 23 comporte deux branches élastiques symétriques, de formes semi-circulaires et qui se terminent chacune par une partie recourbée vers l'extérieur, destinée à transmettre à ces branches élastiques des forces permettant de les écarter l'une de l'autre, lors de l'introduction latérale de l'élément fixe axial 4 d'entrée et de sortie entre elles.

Comme on peut le constater, tous les éléments de positionnement et de guidage des parties fixe et tournante de l'enceinte tubulaire de centrifugation 1 sont solidaires du support 18, en sorte que la précision est fonction de la précision du support 18 lui-même, qui peut être fabriqué avec des tolérances très faible, d'autant plus qu'il ne s'agit pas d'une pièce compliquée à fabriquer. Les autre facteurs qui contribuent à garantir une grande précision sont la distance axiale relativement grande, due à la forme tubulaire allongée de l'enceinte de centrifugation, entre le guidage inférieur et le guidage supérieur. Enfin, le fait de travailler sur une surface cylindrique de guidage 1b de petit diamètre permet de réduire, d'une part les erreurs due au retrait de la matière plastique injectée dans laquelle les enceintes de centrifugation 1, 2 sont fabriquées, le retrait étant proportionnel à la dimension, contrairement à ce que l'on a dans le cas d'une pièce usinée et d'autre part les erreurs de mal rond.

Cette précision du guidage de l'enceinte tubulaire de centrifugation permet de former des écoulements de très faible épaisseur sur la paroi latérale de cette enceinte de centrifugation. Ceci, permet donc d'avoir un faible volume de liquide séjournant dans l'enceinte, ce qui constitue un facteur apte à réduire le risque d'hémolyse et le risque d'activation des plaquettes, ce risque étant certes fonction des forces appliquées, mais aussi du temps pendant lequel les composants du sang sont soumis à ces forces. C'est ainsi que l'on ne peut pas fixer un seuil de force, puisque pour une force donnée, le risque d'hémolyse peut être pratiquement nul pour une certaine durée, alors qu'il peut être beaucoup plus important avec la même force, mais pour une durée sensiblement plus longue.

De préférence, les enceintes tubulaires de centrifugation auront un diamètre compris entre 10 et 40 mm, de préférence 22 mm et seront entraînées à une vitesse de rotation comprise entre 5000 et 100'000 t/min, de sorte que la vitesse tangentielle à laquelle le liquide est soumis n'excède pas 26 m/s. La longueur axiale de l'enceinte tubulaire de centrifugation est comprise avantageusement entre 40 et 200 mm, de préférence 80 mm. De tels paramètres permettent d'assurer un débit de liquide compris entre 20 et 400 ml/min (notamment pour la dialyse), de préférence 60 ml/min, correspondant à un temps de séjour du liquide de 5 à 60 s, de préférence 15 s dans l'enceinte tubulaire.

Nous allons examiner maintenant plus en détail la conception de l'enceinte tubulaire de centrifugation 1 destinée à être associée au séparateur centrifuge qui vient d'être décrit. On peut préciser ici que tout ce qui a été expliqué dans la description qui précède, en ce qui concerne les dimensions, l'entraînement, le positionnement et le guidage de l'enceinte tubulaire de centrifugation 1 s'applique également à l'enceinte tubulaire de centrifugation 2. Par contre, cette dernière ne comportant qu'une sortie 24 pour le PPP, est intérieurement de conception plus simple que l'enceinte tubulaire 1.

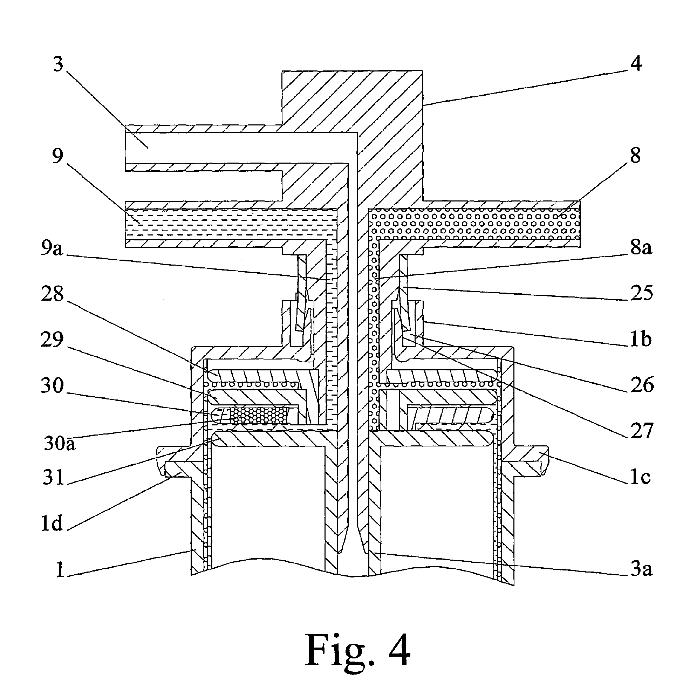

Comme illustré par la figure 4, l'enceinte tubulaire 1 est réalisée à partir de deux parties qui se terminent par des collerettes annulaires respectives 1c, 1d soudées l'une à l'autre. L'espace interne de l'enceinte est délimité par la paroi essentiellement cylindrique de cette enceinte. L'élément fixe axial 4 d'entrée et de sortie pénètre dans cette enceinte tubulaire 1 par une ouverture axiale ménagée à travers l'élément cylindrique de guidage axial 1b. L'étanchéité entre cette ouverture axiale solidaire de l'enceinte entraînée en rotation et l'élément fixe axial 4 est réalisée par un joint tubulaire 25 dont un segment est fixé sur une portion cylindrique de cet élément fixe axial 4 d'entrée et de sortie, tandis qu'un autre segment est introduit dans un espace annulaire 26 de l'élément cylindrique de guidage axial 1b et prend appui sur une surface convexe de la paroi tubulaire 27 séparant l'ouverture axiale traversant l'élément cylindrique de guidage axial 1b de l'espace annulaire 26. Cette étanchéité sert à préserver la stérilité du liquide contenu dans l'enceinte de centrifugation. Comme illustré sur cette figure 4, la partie du joint tubulaire 25 qui prend appui sur la paroi tubulaire 27 subit une légère déformation radiale pour assurer l'étanchéité.

On peut constater que le diamètre sur lequel frotte le joint tubulaire 25 est petit et est de préférence < 10 mm, en sorte que l'échauffement est limité à des valeurs acceptables. On peut constater d'après les dimensions possibles susmentionnées données pour l'enceinte tubulaire de centrifugation, que la distance axiale entre les moyens de centrage et de guidage supérieur et inférieur de cette enceinte, est supérieure à cinq fois le diamètre de l'élément cylindrique de guidage axial 1b. Compte tenu de la précision avec laquelle l'enceinte tubulaire 1 est guidée et la précision que peut atteindre le positionnement relatif de l'élément fixe axial 4 d'entrée et de sortie, le joint n'a pratiquement pas à compenser de défaut de concentricité de l'enceinte tubulaire 1 en rotation, comme c'est le cas des dispositifs susmentionnés de l'état de la technique travaillant en écoulement semi-continu. Ceci contribue également à réduire l'échauffement du joint tubulaire tournant 25 et permet donc d'augmenter la vitesse de rotation de l'enceinte tubulaire de centrifugation.

L'élément fixe axial 4 d'entrée et de sortie comporte une partie tubulaire 3a qui prolonge le conduit d'alimentation 3 connecté à cet élément fixe axial 4 jusqu'à proximité du fond de l'enceinte tubulaire de centrifugation 1 pour y amener le sang ou un autre liquide physiologique à séparer.

Les conduits de sortie 8 et 9 connectés à l'élément fixe axial 4 d'entré et de sortie comportent chacun un segment axial 8a, respectivement 9a qui pénètre dans l'enceinte tubulaire et débouche dans la partie de l'élément fixe axial 4 d'entrée et de sortie qui se situe au voisinage de l'extrémité supérieure de l'enceinte tubulaire de centrifugation 1. L'extrémité d'admission de chacun de ces conduits de sortie 8a, 9a est formée par une fente circulaire. Chacune de ces fentes est ménagée entre deux disques 28, 29, respectivement 30, 31, solidaires de l'élément fixe axial 4 d'entrée et de sortie.

Dans cet exemple, la distance radiale entre les bords des disques 28, 29 et la paroi latérale de l'enceinte 1 est inférieure à la distance radiale entre les bords des disques 30, 31 et cette même paroi latérale. Par cette disposition, le plasma riche en plaquettes PRP de densité plus faible que les globules rouges RBC est aspiré par le dispositif de pompage 13 (fig. 1) dans le conduit de sortie 9, alors que les globules rouges sont aspirées par le gradient de pression engendré par la force centrifuge au sein du liquide, dans le conduit de sortie 8.

Comme on peut le constater, le diamètre de la partie de l'enceinte tubulaire de centrifugation 1 située dans la zone de sortie du PRP et des RBC où se situent les disques 28 à 31 est légèrement plus grand que celui du reste de cette enceinte tubulaire 1, de manière à augmenter les épaisseurs respectives des couches de PRP et de RBC pour faciliter leurs sorties séparées.

Un espace mort est ménagé entre les disques adjacents 29 et 30. Il a pour rôle de piéger les leucocytes, dont la densité se situe entre celle des RBC et des plaquettes, mais dont la taille est très supérieure à celle des RBC et des plaquettes. Le disque 30 comporte un filtre 30a pour permettre de séparer les leucocytes du plasma et de ne piéger dans l'espace mort entre les disques 29 et 30 que les leucocytes.

La deuxième forme d'exécution de l'enceinte tubulaire de centrifugation illustrée par la figure 5 se différencie de celle de la figure 4 essentiellement par la présence d'un barrage 32. Celui-ci présente une forme annulaire, comprenant une partie cylindrique 32a située en face de l'ouverture d'admission circulaire du PRP ménagée entre les disques 30 et 31. Le diamètre de cette partie cylindrique 32a est choisi pour se situer dans l'espace séparant les bords des disques 28, 29 de la paroi latérale de l'enceinte 1 correspondant sensiblement à l'interface entre les couches formées par les RBC et le PRP. Les deux extrémités de cette partie cylindrique 32a se terminent par des anneaux plans, 32b, 32c. L'anneau plan 32b s'étend à l'extérieur de la partie cylindrique 32a, tandis que l'anneau plan 32c s'étend à l'intérieur de cette partie cylindrique 32a. L'anneau plan externe 32b est logé dans un dégagement de la collerette annulaire 1d et est pincé entre les deux collerettes annulaires 1c et 1d. Cet anneau plan externe 32b est encore traversé par une pluralité d'ouvertures 32d pour permettre le passage des RBC.

Ce barrage 32 a trois rôles à jouer. L'un est de créer une barrière physique entre l'ouverture d'admission circulaire du PRP située entre les disques 30 et 31 et les RBC, de manière à éviter que les remous engendrés par l'aspiration au niveau de l'ouverture d'admission ne risque de re-mélanger les RBC et le PRP. Un deuxième rôle est de permettre de collecter les RBC sur le même diamètre que le plasma, ce qui diminue l'hémolyse du fait que les bords des disques 30, 31 formant l'ouverture de sortie des RBC trempent moins profondément dans la couche de RBC, puisque tous les disques 28 à 31 sont de même diamètre. Enfin, le troisième rôle est de retenir au moins partiellement les leucocytes à l'intérieur de la partie cylindrique 32a du barrage 32.

Le reste de cette enceinte tubulaire de centrifugation 1 selon cette deuxième forme d'exécution est pratiquement semblable à la première forme d'exécution qui vient d'être décrite. Un filtre à déleucocyter semblable au filtre 29a de la figure 4 peut également être prévu pour piéger les leucocytes entre les disques 29 et 30.

## Revendications

1. Séparateur centrifuge pour un fluide physiologique, en particulier pour le sang, comprenant un conduit fixe (3) pour alimenter en continu une enceinte jetable (1) rigide de centrifugation en liquide à séparer, deux conduits fixe de sortie (8, 9) dont les ouvertures d'admission respectives se situent dans deux zones de concentration des constituants séparés pour les retirer en continu, ces conduits fixes (3, 8, 9) étant solidaires d'une partie fixe (4) de ladite enceinte (1), des moyens de guidage en rotation (21) de cette enceinte (1), des moyens d'entraînement de support et de guidage (20a) en prise avec un élément d'accouplement axial (1a) solidaire du fond de ladite enceinte (1) et des moyens de positionnement (23) de la partie fixe de ladite enceinte (1) par rapport auxdits moyens d'entraînement de support et de guidage, **caractérisé en ce qu'**il comporte d'autre part des éléments de guidage rotatifs (21) pour venir en prise avec une surface cylindrique (1b) coaxiale audit axe de rotation solidaire de l'extrémité supérieure de ladite enceinte, au moins un desdits éléments de guidage rotatifs (21) étant monté de manière à pouvoir être écarté dudit axe de rotation à l'encontre d'un moyen élastique de rappel pour permettre l'introduction et l'enlèvement de ladite enceinte (1).

2. Séparateur selon la revendication 1, dans lequel ladite enceinte de centrifugation (1) est tubulaire et présente un diamètre compris entre 10 et 80 mm et la vitesse à laquelle elle est entraînée est compris entre 3000 et 100'000 t/min pour que la vitesse tangentielle appliquée auxdits constituants soit inférieure à 26 m/s.

3. Séparateur centrifuge selon l'une des revendications précédentes, comportant d'une part un arbre d'entraînement (20a), de support et de guidage, coaxial à l'axe de rotation de ladite enceinte (1), conformé pour venir en prise avec un élément d'accouplement axial (1a) solidaire du fond de ladite enceinte (1), d'autre part au moins trois galets rotatifs de guidage (21) répartis autour dudit axe de rotation et à égales distances de celui-ci, pour venir en prise avec une surface cylindrique (1b) solidaire de l'extrémité supérieure de ladite enceinte (1).

4. Séparateur selon la revendication 3, dans lequel ladite surface cylindrique (1b) avec laquelle les galets de guidage rotatifs (21) viennent en prise présente un diamètre sensiblement plus faible que celui de l'enceinte de centrifugation.

5. Séparateur selon la revendication 1, dans lequel le côté desdits moyens de guidage rotatifs (21) pour venir en prise avec une surface de révolution coaxiale audit axe de rotation solidaire de l'extrémité supérieure de ladite enceinte dirigé vers le fond de ladite enceinte est adjacent à une portée de ladite enceinte (1) pour limiter la liberté de déplacement axial de celle-ci.

6. Séparateur selon la revendication 3, dans lequel l'axe de pivotement d'au moins un desdits cylindres de guidage (21) présente une inclinaison < 2° dans un plan tangent à un cercle centré sur l'axe de rotation de ladite enceinte (1).

7. Séparateur selon la revendication 1, dans lequel la distance axiale entre l'extrémité d'entraînement et de guidage dudit arbre d'entraînement (20a) et lesdits cylindres de guidage (21) est au moins égale à 5 fois la distance séparant les cylindres de guidage (21) de l'axe de rotation de ladite enceinte (1) de centrifugation.

8. Séparateur selon l'une des revendications précédentes, dans lequel lesdits moyens d'entraînement de support et de guidage (20a) et lesdits moyens de guidage rotatifs (21) sont solidaires d'un support commun (18) relié au bâti du séparateur par une suspension anti-vibrations (19).

## Claims

1. A centrifugal separator for a physiological fluid, particularly blood, comprising a fixed conduit (3) which continuously supplies a rigid disposable centrifugation chamber (1) with the liquid to be separated, two fixed outlet conduits (8, 9) whose respective inlet ports are located in two zones of concentration of the separated constituents in order to remove them continuously, these fixed conduits (3, 8, 9) being integrally connected to a fixed part (4) of said chamber (1), means (21) for guiding this chamber (1) in rotation, means (20a) for driving, supporting and guiding in engagement with an axial coupling element (1a) integrally connected to the bottom of said chamber (1), and means (23) for positioning the fixed part of said chamber (1) relative to said means for driving, supporting and guiding, **characterized in that** it further comprises rotary guide means (21) for engaging with a cylindrical surface (1b) that is coaxial with respect to said axis of rotation and integrally connected to the upper end of said chamber, at least one of said rotary guide rollers (21) being mounted in such a way as to be able to be moved away from said axis of rotation against an elastic return means in order to permit the introduction and removal of said chamber (1).

2. The separator as claimed in claim 1, in which said centrifugation chamber (1) is tubular and has a diameter of between 10 and 80 mm, and the speed at which it is driven is between 3000 and 100,000 rpm, such that the tangential speed applied to said constituents is less than 26 m/s.

3. The centrifugal separator as claimed in one of the preceding claims, comprising, on the one hand, a drive shaft (20a), for supporting and guiding, which is coaxial with respect to the axis of rotation of said chamber (1) and is designed to engage with an axial coupling element (1a) integrally connected to the bottom of said chamber (1) and, on the other hand, at least three rotary guide rollers (21) distributed about said axis of rotation and at equal distances from the latter, for engaging with a cylindrical surface (1b) integrally connected to the upper end of said chamber (1).

4. The separator as claimed in claim 3, in which said cylindrical surface (1b) with which the rotary guide rollers (21) engage has a diameter substantially smaller than that of the centrifugation chamber.

5. The separator as claimed in claim 1, in which the side of said rotary guide means (21) for engaging with a surface of revolution coaxial with respect to said axis of rotation and integral with the upper end of said chamber directed toward the bottom of said chamber is adjacent to a surface area of said chamber (1) for limiting the freedom of axial displacement thereof.

6. The separator as claimed in claim 3, in which the pivot axis of at least one of said guide cylinders (21) has an inclination of < 2° in a plane tangential to a circle centered on the axis of rotation of said chamber (1).

7. The separator as claimed in claim 1, in which the axial distance between the driving and guiding end of said drive shaft (20a) and said guide cylinders (21) is at least equal to 5 times the distance separating the guide cylinders (21) from the axis of rotation of said centrifugation chamber (1).

8. The separator as claimed in one of the preceding claims, in which said means (20a) for driving, supporting and guiding and said rotary guide means (21) are integrally connected to a common support (18) linked to the framework of the separator via an anti-vibration suspension (19).

## Patentansprüche

1. Zentrifugalseparator für eine physiologische Flüssigkeit, insbesondere für Blut, der über eine feste Leitung (3), um einem steifen Einwegzentrifugalbehälter (1) kontinuierlich zu trennende Flüssigkeit zuzuführen, über zwei feste Auslassleitungen (8, 9), bei denen die jeweiligen Einlassöffnungen in zwei Konzentrationsbereichen von getrennten Bestandteilen angeordnet sind, um diese kontinuierlich abzuleiten, wobei die festen Leitungen (3, 8, 9) einstückig mit einem festen Teil (4) des Behälters (1) ausgebildet sind, über Drehführungsmittel (21) des Behälters (1), über Antriebsmittel zum Stützen und Führen (20a), die mit einem axialen Kupplungselement (1a) in Eingriff sind, das einstückig mit der Rückseite des Behälters (1) ausgebildet ist, und über Positionierungsmittel (23) des festen Teils des Behälters (1) in Bezug auf die Antriebsmittel zum Stützen und Führen verfügt, **dadurch gekennzeichnet, dass** er außerdem drehbare Führungselemente (21) aufweist, um mit einer zylindrischen Oberfläche (1 b) in Eingriff zu kommen, die koaxial zu der Drehachse und einstückig mit dem oberen Ende des Behälters ist, wobei wenigstens eines der drehbaren Führungselemente (21) so angebracht ist, dass es von der Drehachse entfernbar gegenüber einem elastischen Rückzugsmittel ist, um das Einsetzen und Entfernen des Behälters (1) zu gestatten.

2. Separator nach Anspruch 1, bei dem der Zentrifugalbehälter (1) röhrenartig ist sowie einen Durchmesser zwischen 10 und 80 mm aufweist und wobei die Geschwindigkeit, in der er angetrieben wird, zwischen 3000 und 100.000 U/min beträgt, damit die auf die Bestandteile angewandte Tangentialgeschwindigkeit kleiner als 26 m/s ist.

3. Zentrifugalseparator nach einem der vorangehenden Ansprüche, der zum einen über eine Antriebswelle (20a) zum Stützen und Führen, die koaxial zu der Drehachse des Behälters (1) und dazu eingerichtet ist, mit einem axialen, einstückig mit der Rückseite des Behälters (1) ausgebildeten Kupplungselement (1a) in Eingriff zu kommen, sowie zum anderen über wenigstens drei drehbare Führungsrollen (21) verfügt, die um die Drehachse herum und in gleichem Abstand von dieser angeordnet sind, um mit einer zylindrischen, einstückig mit dem oberen Ende des Behälters (1) ausgebildeten Oberfläche (1 b) in Eingriff zu kommen.

4. Separator nach Anspruch 3, bei dem die zylindrische Oberfläche (1 b), mit der die drehbaren Führungsrollen (21) in Eingriff kommen, einen wesentlich kleineren Durchmesser als den des Zentrifugalbehälters aufweist.

5. Separator nach Anspruch 1, bei dem die Seite der drehbaren Führungsmittel (21), um mit einer koaxialen Umdrehungsfläche zu der einstückig mit dem oberen Ende des Behälters ausgebildeten Drehachse in Eingriff zu kommen, die in Richtung der Rückseite des Behälters ausgerichtet ist, benachbart in Reichweite des Behälters (1) angeordnet ist, um dessen axiale Verschiebefreiheit zu begrenzen.

6. Separator nach Anspruch 3, bei dem die Schwenkachse wenigstens eines Führungszylinders (21) eine Neigung < 2° in einer Tangentialebene zu einem mittig zu der Drehachse des Behälters (1) ausgerichteten Kreis aufweist.

7. Separator nach Anspruch 1, bei dem der axiale Abstand zwischen dem Antriebs- und Führungsende der Antriebswelle (20a) und den Führungszylindern (21) wenigstens dem fünffachen Abstand entspricht, der die Führungszylinder (21) der Drehachse des Zentrifugalbehälters (1) trennt.

8. Separator nach einem der vorangehenden Ansprüche, bei dem die Antriebsmittel zum Stützen und Führen (20a) und die drehbaren Führungsmittel (21) einstückig mit einem gemeinsamen Träger (18) ausgebildet sind, der mit dem Separatorgehäuse durch eine Antivibrationsaufhängung (19) verbunden ist.
